# EUROPÄISCHE PATENTSCHRIFT

(11) **EP 1 936 524 B1**
(45) Veröffentlichungstag und Bekanntmachung des Hinweises auf die Patenterteilung: **21.03.2018**
(21) Anmeldenummer: 06126930.4
(22) Anmeldetag: 21.12.2006
(51) Int. Cl.: G06F 19/00

(54) **Blutbehandlungseinrichtung**
Blood treatment device
Dispositif de traitement de sang

(43) Veröffentlichungstag der Anmeldung: 25.06.2008
(73) Patentinhaber: B. Braun Avitum AG, 34212 Melsungen (DE)
(72) Erfinder: Moll, Stefan, 34212 Melsungen (DE); Bock, Gerhardt, 36289 Friedewald (DE); Sándor, Dolgos, H-2000 Szentendre (HU)
(74) Vertreter: Winter, Brandl, Fürniss, Hübner, Röss, Kaiser, Polte - Partnerschaft mbB

(56) Entgegenhaltungen:
- EP-A1- 1 535 576
- WO-A-02/26291
- WO-A2-02/11049

## Beschreibung

Die Erfindung betrifft eine Blutbehandlungseinrichtung zur extrakorporalen Blutbehandlung, mit mindestens einem Blutbehandlungsgerät, welches aufweist einen Filter, der an einen Blutkreislauf und einen Dialysatkreislauf angeschlossen ist, eine Steuereinrichtung zur Steuerung von Funktionsabläufen, ein Anwender-Interface, das einen Bildschirm und Eingabemittel aufweist.

Die bekannten Blutbehandlungseinrichtungen, wie beispielsweise Dialysegeräte, sind in Aufbau und Bedienung komplex, weil sie zahlreiche Möglichkeiten der Einstellung und des Programmablaufs bieten. Daher muss das Bedienungspersonal, im Folgenden als "Anwender" bezeichnet, besonders geschult sein. Die Behandlungsart, die mit einem Blutbehandlungsgerät durchgeführt werden kann, umfasst diverse Phasen. Hierzu gehören beispielsweise: Vorbereitung des Gerätes, das Anlegen des Patienten, d.h. das Verbinden des Patienten mit dem Schlauchsystem des Gerätes, die eigentliche Therapie, z.B. die Blutreinigung, und die Nachbereitung der Therapie, beispielsweise durch Zuführen bestimmter Medikamente.

Bei den Blutbehandlungsgeräten gibt es zwei unterschiedliche Strategien: Die erste Strategie besteht darin, dass der Anwender den jeweils nächsten Schritt des Behandlungsvorgangs aufgrund von Erfahrungen am Gerät auswählt. Die zweite Strategie sieht einen automatischen Programmablauf vor, wobei das Blutbehandlungsgerät mittels Sensoren den augenblicklichen Zustand eines Programmablaufs feststellt und nach Beendigung eines Programmschritts zwangsweise den nächsten Programmschritt ausführt.

DE 103 23 843 A1 beschreibt ein Konzept, bei dem auf einem Touchscreen verschiedene zeitliche Modi einer Blutbehandlung dargestellt werden, von denen der Anwender eine Auswahl treffen kann. Der jeweils laufende zeitliche Modus wird von einer Steuereinrichtung identifiziert und auf dem Touchscreen ausgewählt dargestellt. Das Ende eines zeitlichen Modus wird festgestellt, um automatisch den Beginn des anschließenden zeitlichen Modus einzuleiten. Hierbei werden die Modi in fester zeitlicher Folge abgearbeitet. Sobald die Sensoren einen bestimmten Modus erfassen, erfolgt der automatische Übergang zum nächsten Modus. Dabei wird eine starre Funktionsabfolge realisiert, wobei der Bediener keine Möglichkeit hat, das vorgespeicherte Programm zu verändern. Dies stellt eine starke Einschränkung in der Flexibilität dar, da das Dialysegerät einem vermeintlich idealen Ablauf automatisch und zwanghaft folgt. Etwaige Sondersituationen, wie sie bei unterschiedlichen Patienten immer wieder auftreten, können nicht berücksichtigt werden. Der feste Programmablauf bringt den Anwender in ein starres System.

In WO 02/26291 A1 ist eine Dialysemaschine beschrieben, die an einen Mikroprozessor für die Hauptkontrolle und einen weiteren Mikroprozessor für die Funktionalitätsprüfung der Dialysemaschine ausweist. Der Mikroprozessor für die Hauptsteuerung enthält mehrere Speicher, die virtuell separiert und operationell unabhängig voneinander sind. Diese Speicher liefern Redundanz der Speicherung. Jeder dieser Speicher speichert die Dialysebehandlungsparameter, wie Änderungen der Ultrafiltration während der Dialysebehandlung, die Variation der Konzentration der Dialyseflüssigkeit während der Dialysebehandlung, usw.

Aus WO02/11049 A2 ist eine medizinische Pumpe mit einer Programmbibliothek zur Steuerung der Pumpe bekannt.

Eine fest vorgegebene Abfolge der einzelnen Schritte stellt eine starke Einschränkung in der Flexibilität dar. Das Blutbehandlungsgerät folgt zwanghaft einem vermeintlich idealen Ablauf, der automatisch durchgeführt wird. Etwaige Sondersituationen, wie sie bei unterschiedlichen Patienten auftreten, oder landesspezifische Abläufe, die durch lokale Gewohnheiten oder Gesetzesanforderungen entstehen, oder auch klinikspezifische Abläufe können damit nur unzureichend berücksichtigt werden.

Um eine individuellere aber dennoch automatisierte Gestaltung des Funktionsablaufs des Gerätes zu gewähren, sind einige Hersteller dazu übergegangen, einzelne Abläufe auswählbar zu gestalten. So kann z.B. entschieden werden, ob am Therapieende der benutzte Dialysator sofort und automatisch entleert wird, oder ob der Entleervorgang erst nach der manuellen Aktivierung über eine bestimmte Taste beginnt. Ähnliches gilt für das Entleeren der Bicarbonat-Kartusche oder die potentiell folgende Desinfektion.

Die Auswahlmöglichkeit bzw. Aktivierung der Funktionalität erfolgt aus Sicherheitsgründen entweder nur durch einen Servicetechniker oder nur in gesondert zugänglichen Menüs. Nachteil dieser Lösung ist, dass die einzelne Auswahl des Verhaltens bei bestimmten Schritten für das Pflegepersonal unverständlich ist und damit der Überblick für den Gesamtablauf verloren geht. Ferner ist eine Abstimmung zwischen Schwester, Arzt und Techniker am Gerät nötig. Der an einzelnen Stellen modifizierte Ablauf ist dann nicht individuell anpassbar an Tage, Patienten und Verbrauchsmaterial und nur auf dem dafür eingerichteten Blutbehandlungsgerät installiert.

Der Erfindung liegt die Aufgabe zugrunde, eine Blutbehandlungseinrichtung zu schaffen, mit der der Anwender die Möglichkeit hat, den gesamten Behandlungsablauf auf spezielle Bedürfnisse anzupassen, wie beispielsweise den Behandlungsstandard eines Dialysezentrums, das vorhandene Verbrauchsmaterial oder die Anforderungen des Patienten. Hierbei soll für den Anwender der Überblick über den gesamten Funktionsablauf der Behandlung erhalten bleiben und es soll gewährleistet sein, dass für den Patienten keine unsinnigen oder gefährdenden Einstellungen entstehen. Eine weitere Aufgabe ist die Schaffung einer Blutbehandlungseinrichtung, bei der die Gestaltung des gesamten Funktionsablaufs vom Einschalten des Geräts bis zum Desinfizieren des Schlauchsystems und der Gerätekomponenten am Blutbehandlungsgerät und/oder an einem separaten Rechner erfolgen kann.

Die erfindungsgemäße Blutbehandlungseinrichtung ist durch den Patentanspruch 1 definiert. Sie ist dadurch gekennzeichnet,
dass eine Programmbibliothek vorgesehen ist, die unterschiedliche Funktionsabläufe für den Betrieb des Blutbehandlungsgerätes in Form von Datensätzen enthält, wobei jeweils ein für einen Behandlungsablauf zu aktivierender Datensatz selektierbar ist, und dass zum Generieren eines in die Programmbibliothek aufzunehmenden Funktionsablaufs eine Einrichtung vorgesehen ist, die eine Bildschirmfläche generiert, welche einen vom Anwender interaktiv zu beantwortenden Fragenkatalog enthält, wobei, wenn die Funktionen, die in den Funktionsablauf zu übernehmen sind, ausgewählt sind, die Folgeinformationen für den Funktionsablauf erzeugt und angezeigt werden, wobei die Folgeinformationen eine Vorbereitungszeit, einen Verbrauch von Flüssigkeit oder notwendige Anwenderreaktionen beinhalten..

Erfindungsgemäß ist eine Programmbibliothek vorgesehen, bei der die darin enthaltenen Programme entweder vorgegeben sind oder vom Anwender, ggf. in Verbindung mit einem Berater, erstellt werden können. Zu diesem Zweck ist ein Eingabeprogramm vorgesehen, das Fragen über den beabsichtigten Funktionsablauf enthält, so dass anhand der Bildschirmoberfläche Parameter eingegeben werden können, aus denen im Computer dann der Funktionsablauf und der zugehörige Datensatz erstellt werden. Damit können Funktionsabläufe bezogen auf den Tag, auf bestimmtes Verbrauchsmaterial wie Schläuche, Dialysat oder Zusatzmedikamente und bezogen auf den Patienten, erstellt werden. Unter einem Funktionsablauf kann der gesamte Therapieablauf zu verstehen sein oder auch ein Teil des Therapieablaufs. Im zweiten Fall können standardisierte Teile zu einem vollständigen Funktionsablauf zusammengesetzt werden.

Ferner ist vorgesehen, dass zum Erzeugen eines in die Programmbibliothek aufzunehmenden Funktionsablaufs eine Einrichtung vorgesehen ist, die eine Bildschirmfläche generiert, welche einen vom Anwender interaktiv zu beantwortenden Fragenkatalog enthält. Das Eingabeprogramm zeigt verschiedene Wahlmöglichkeiten an und gibt ferner die daraus resultierenden Konsequenzen an. Auf diese Art kann durch den Anwender, ggf. unterstützt durch einen Servicetechniker, ein individueller Funktionsablauf erstellt und in die Programmbibliothek eingestellt werden, Für das Pflegepersonal wird die Bedienung des Blutbehandlungsgerätes auf die unbedingt notwendigen Bedienschritte reduziert.

Der Funktionsablauf enthält eindeutige (nicht von Bedingungen abhängige) Entscheidungen, wie beispielsweise das automatische Einschalten des Gerätes am Morgen: Ja/nein. Daneben können auch Abläufe definiert werden, die von bestimmten Bedingungen abhängen, beispielsweise: Wenn Schwester A das Gerät bedient, soll die Desinfektion nicht automatisch starten, jedoch kann bei Schwester B die Desinfektion automatisch starten, Zusätzliche Bedingungen, die den Funktionsablauf beeinflussen, sind beispielsweise der zu behandelnde Patient, dessen Kennung in den Rechner des Blutbehandlungsgeräts eingegeben wird, die Person des Anwenders, dessen Kennung ebenfalls in den Rechner eingegeben wird, beispielsweise durch einen Transponder oder eine maschinenlesbare Personalkarte. Ferner können die Zeit (Uhrzeit oder Kalendertag) oder die verwendeten Verbrauchsmaterialien, die durch einen Transponder eingelesen werden können, die Auswahl des Funktionsablaufs beeinflussen.

Wenn die Funktionen, die in den Funktionsablauf zu übernehmen sind, ausgewählt sind, werden im Computer die Konsequenzen der Auswahl ermittelt und angezeigt. Dies können sein: Die Dauer der Vorbereitungszeiten, die Höhe des Verbrauchs an Dialysat oder notwendiger Anwenderaktionen.

Nachdem ein Funktionsablauf generiert wurde, erzeugt das Vorbereitungsprogramm daraus ein für das Blutbehandlungsgerät lesbares Set von Konfigurationsdaten. Dieses Konfigurationsdatenset wird mit einer Identifikation versehen und ist dann auf jedem der zu der Blutbehandlungseinrichtung gehörenden Blutbehandlungsgeräte verwendbar. Somit können die Datensätze unterschiedlicher Funktionsabläufe auf die Steuereinrichtungen mehrerer Blutbehandlungsgeräte kopiert werden.

Aus dem am Computer beantworteten Fragenkatalog wird automatisch der aus den Konfigurationsdaten bestehende Datensatz erstellt. Die Datensätze können per Datenträger oder per Netzwerkverbindung auf dem Blutbehandlungsgerät installiert werden. Dies kann einmalig oder permanent geschehen. Dabei können mehrere unterschiedliche Datensätze in einem Blutbehandlungsgerät gespeichert sein, wobei zu Beginn oder während der Therapie der zu aktivierende Datensatz ausgewählt wird. Die Auswahl des Datensatzes kann mit Hilfe des Bildschirms durch den Anwender erfolgen oder auch automatisch, z.B. in Abhängigkeit von einer Patientenkarte oder einer maschinenlesbaren Identifizierung des Anwenders, sowie in Abhängigkeit von der gewählten Therapieform oder den verwendeten Verbrauchsmaterialien,

Dies ermöglicht es, dass eine Therapie einen individuellen, aber zuvor definierten und optimierten, Ablauf enthält. Falls gewünscht, ist es sogar möglich, jedem Anwender (Krankenschwester) einen individuell bevorzugten Bedienungsablauf zuzuordnen oder besonders kritischen Patienten einen Ablauf zuzuordnen, der wenig Automatismen beinhaltet und somit eine hohe Präsenz und Aufmerksamkeit der Schwester bedingt.

Zu den Verbrauchsartikeln des Blutbehandlungsgeräts gehört neben dem Schlauchsystem auch der Filter oder Dialysator. Wenn der Steuereinrichtung des Gerätes die Verbrauchsartikel nach Typ und Größe bekannt sind, so kann in Abhängigkeit von den eingesetzten Verbrauchsartikeln der Spül- und Füllvorgang gewählt werden. Beispielsweise wird man bei "High flux Dialysatoren" einen anderen Spül- und Füllvorgang wählen als bei "Low flux Dialysatoren". Dadurch werden Zeit und Spülflüssigkeit gespart.

In der Regel reicht es aus, eine geringe Zahl von Datensätzen für ein Dialysezentrum oder den Betreiber mehrerer Dialysezentren zur Verfügung zu stellen und die entsprechenden Datensätze auf allen Blutbehandlungsgeräten zur Verfügung zu stellen. Vor Ort kann dann der anzuwendende Datensatz in Abhängigkeit von Sensorsignalen und/oder durch manuelle Betätigung bzw. Eingabe ausgewählt werden.

Es ist anzustreben, dem erfahrenen Anwender eine größere Anzahl von Datensätzen und somit Funktionsabläufen zur Verfügung zu stehen als der normalen Schwester. Letztere erhält eine eingeschränkte Wahlmöglichkeit. Auf diese Weise können sehr spezifische und optimierte Behandlungsabläufe verwendet werden, ohne die Gefahr von Fehlbedienungen durch normales Pflegepersonal.

Die Erfindung betrifft ferner ein Verfahren zum Betrieb eines Blutbehandlungsgerätes nach Patentanspruch 15.

Vorteilhafte Ausgestaltungen und Weiterbildungen der Erfindung sind in den jeweiligen abhängigen Ansprüchen angegeben.

Im Folgenden wird unter Bezugnahme auf die Zeichnungen ein Ausführungsbeispiel der Erfindung näher erläutert.

Es zeigen:
- Fig. 1: eine schematische Darstellung eines Blutbehandlungsgerätes,
- Fig. 2: eine Darstellung der Kooperation eines separaten Rechners mit einer Blutbehandlungseinrichtung, die mehrere Blutbehandlungsgeräte enthält, und
- Fig. 3: den Aufbau eines an einem Touchscreen generierten Fragenkatalogs zum interaktiven Eingeben von Antworten zum Zwecke der Erstellung eines Datensatzes eines Funktionsablaufes eines Blutbehandlungsgerätes.

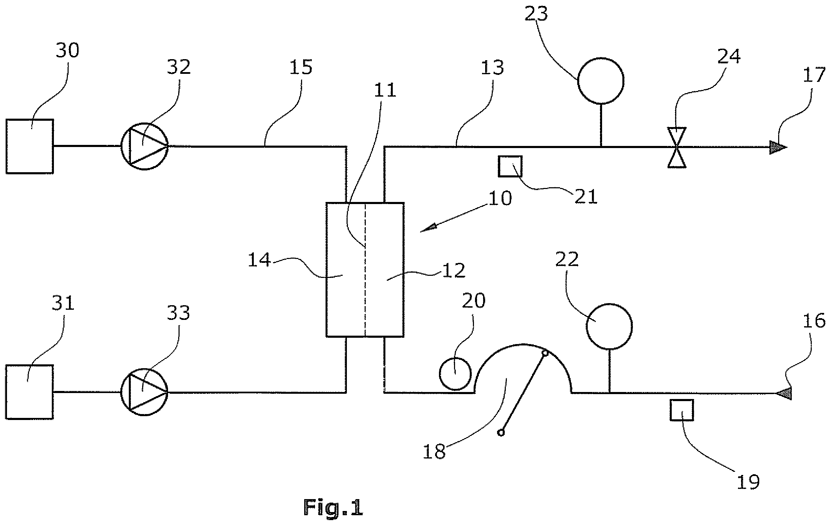

Das in Fig. 1 dargestellte Blutbehandlungsgerät ist ein Dialysegerät mit einem Dialysator 10, der zwei Kammern aufweist, zwischen denen sich eine Dialysatormembran 11 befindet. Die Blutkammer 12 des Dialysators ist an einen Blutkreislauf 13 angeschlossen und die Dialysatkammer 14 ist an einen Dialysatkreislauf 15 angeschlossen. Der Blutkreislauf 13 enthält ein Schlauchsystem mit einem Einlass 16 und einem Auslass 17. Das Schlauchsystem ist durch eine Blutpumpe 18 geführt, die einen volumetrischen Fördervorgang durchführt, wobei die Fließrate proportional der Antriebsgeschwindigkeit ist. Der Blutkreislauf 13 enthält mehrere Sensoren, nämlich einen Blutsensor 19, der anhand der Farbe des Bluts das Vorhandensein von Blut in dem Schlauchsystem feststellt, einen Schlauchsensor 20, der feststellt, ob ein Schlauch ordnungsgemäß in die Blutpumpe 18 eingelegt ist, einen Luftdetektor 21 zum Feststellen des Vorhandenseins von Luft im Schlauchsystem, einen Drucksensor 22 zur Messung des arteriellen Blutdrucks und einen Drucksensor 23 zur Messung des venösen Blutdrucks.

Die Blutpumpe 18 bildet einen im Blutkreislauf vorhandenen Aktor. Ein weiterer Aktor wird von einer in der venösen Leitung enthaltenen Schlauchklemme 24 gebildet.

Der Dialysatkreislauf 15 ist an eine Dialysatquelle 30 und einen Ablauf 31 angeschlossen. Die Vorlaufleitung enthält eine erste Pumpe 32 und die Rücklaufleitung eine zweite Pumpe 33.

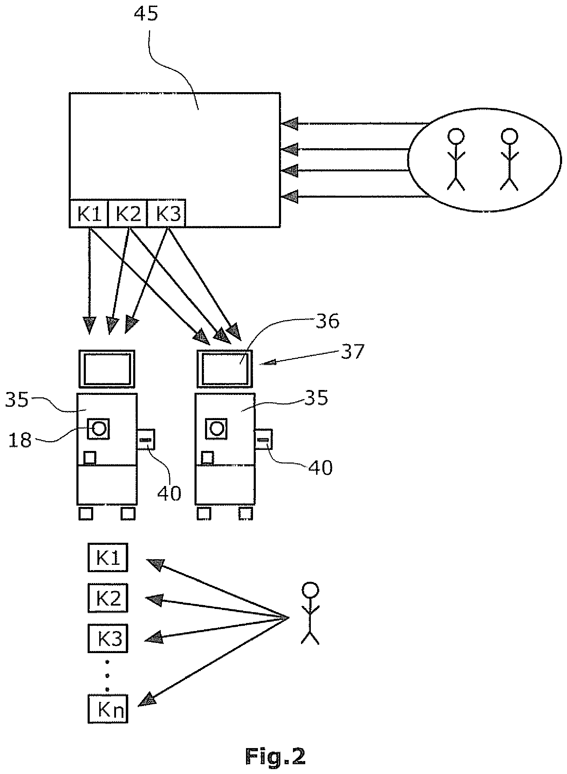

In Fig. 2 sind stellvertretend für eine Mehrzahl von Dialysegeräten zwei Dialysegeräte 35 dargestellt, die zu derselben Dialyseeinrichtung gehören. Jedes Dialysegerät 35 weist an der Vorderseite eines Gehäuses eine Blutpumpe 18 auf. Auf dem Gehäuse ist ein Bildschirm 36 angeordnet, der das Anwender-Interface 37 bildet. Darin befindet sich auch die Steuereinrichtung des Blutbehandlungsgerätes 35.

Jedes Blutbehandlungsgerät ist mit einem Lesegerät 40 versehen, das hier als Kartenleser ausgebildet ist, welcher eine Karte in Form einer Chipkarte lesen kann. Die Chipkarten sind einerseits Anwenderkarten, die eine Identifizierung des jeweiligen Anwenders enthalten, und andererseits Patientenkarten, die eine Identifizierung des jeweiligen Patienten enthalten. Aus der Identifizierung kann die Steuereinrichtung des Gerätes auch Angaben über die Berechtigung bzw. die Behandlungsbedürfnisse der betreffenden Person enthalten. Anstelle der Lesegeräte 40 können beispielsweise auch Transponder-Lesegeräte zum berührungslosen Abrufen von Informationen von einem Transponder eingesetzt werden, der von der betreffenden Person am Körper getragen wird.

In Fig. 2 ist ein externer Rechner 45 dargestellt, der eine Programmbibliothek aus zahlreichen Datensätzen enthält, von denen jeder einem Funktionsablauf entspricht. Die Datensätze K1, K2, K3 werden von dem Rechner 45 an jedes der Blutbehandlungsgeräte geliefert, und zwar entweder drahtlos oder durch ein drahtgebundenes Netz. Die in der Programmbibliothek enthaltenen Programme enthalten neben den Konfigurationsdatensätzen auch Anwenderwünsche und weitere Informationen.

Der separate Rechner 45 enthält einen Touchscreen zur interaktiven Eingabe von Randbedingungen bzw. Anforderungen. Hierzu wird der in Fig, 3 dargestellte Fragenkatalog 50 auf dem Touchscreen angezeigt. Die Kästchen werden vom Anwender bzw. Berater markiert, um die betreffende Bedingung oder Information einzugeben. Aus dem beantworteten Fragenkatalog 50 erstellt der Rechner 45 einen Datensatz Kₙ, der den Blutbehandlungsgeräten 35 zur Verfügung gestellt wird. Dieser Datensatz bildet den Konfigurationsdatensatz, der den Ablauf des Arbeitsprogramms des Blutbehandlungsgerätes 35 angibt.

An dem Blutbehandlungsgerät 35 ist der betreffende Datensatz K1... Kₙ vom Anwender auswählbar oder er wird durch Sensoren bestimmt, die z.B. die Art der verwendeten Verbrauchsartikel (Dialysator, Schlauchsystem usw.) ermitteln, so dass in Abhängigkeit hiervon eine Auswahl von verwendbaren Datensätzen erfolgt. Die endgültige Auswahl eines Datensatzes kann dann in Abhängigkeit von anderen Parametern erfolgen, wie Daten des Anwenders, des Patienten oder auch durch manuelle Auswahl.

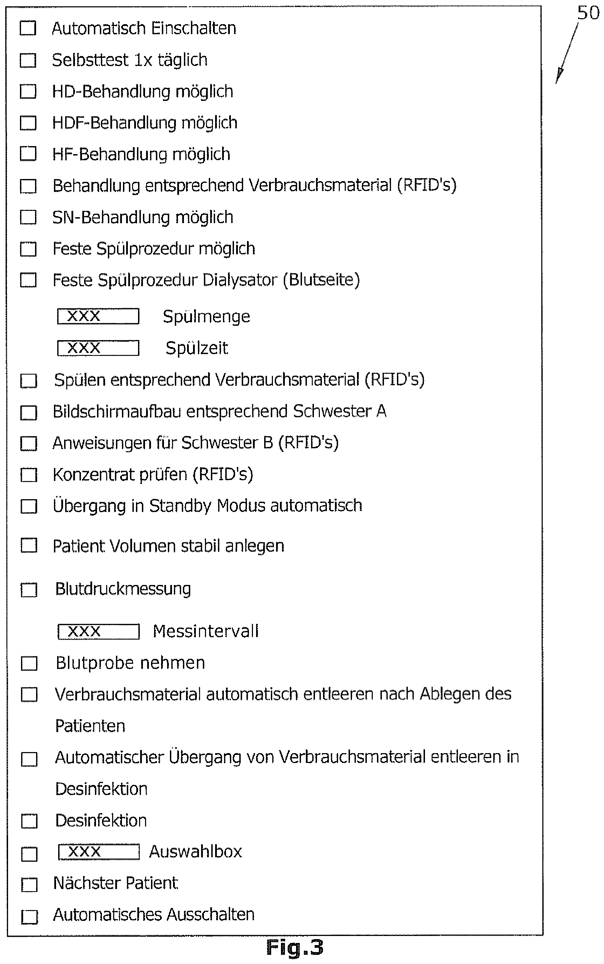

In Fig. 3 bedeutet RFID "radio frequency identification". Es handelt sich um drahtlos abrufbare Identifizierungen, die durch Transponder an einer Abfragestation übermittelt werden.

Der separate Rechner 45 enthält zur Erstellung der Datensätze aus dem Fragenkatalog 50 ein Modell des Blutbehandlungsgerätes 35, in welches Parameter eingebbar sind, und das einen Funktionsablauf mit diesen Parametern simuliert. Im Anschluss an einen derartigen Funktionsablauf wird der betreffende Datensatz in die Programmbibliothek eingespeichert.

Die in dem Blutbehandlungsgerät vorhandenen Sensoren können auch derart ausgebildet sein, dass sie "tags" detektieren und lesen, die an den Verbrauchsartikeln angebracht sind und deren Typ kennzeichnen, damit auf dieser Grundlage die Auswahl des Datensatzes erfolgen kann.

## Patentansprüche

1. Blutbehandlungseinrichtung zur extrakorporalen Blutbehandlung, mit mindestens einem Blutbehandlungsgerät (35), welches aufweist:
einen Filter (10), der an einen Blutkreislauf (13) und einen Dialysatkreislauf (15) angeschlossen ist,
eine Steuereinrichtung zur Steuerung von Funktionsabläufen,
ein Anwender-Interface (37), das einen Bildschirm (36) und Eingabemittel aufweist,
**dadurch gekennzeichnet,**
**dass** eine Programmbibliothek vorgesehen ist, die unterschiedliche Funktionsabläufe für den Betrieb des Blutbehandlungsgerätes (35) in Form von Datensätzen (Kₙ) enthält, wobei jeweils ein für einen Behandlungsablauf zu aktivierender Datensatz selektierbar ist, und dass zum Generieren eines in die Programmbibliothek aufzunehmenden Funktionsablaufs eine Einrichtung vorgesehen ist, die eine Bildschirmfläche generiert, welche einen vom Anwender interaktiv zu beantwortenden Fragenkatalog (50) enthält, wobei, wenn die Funktionen, die in den Funktionsablauf zu übernehmen sind, ausgewählt sind, die Folgeinformationen für den Funktionsablauf erzeugt und angezeigt werden, wobei die Folgeinformationen eine Vorbereitungszeit, einen Verbrauch von Flüssigkeit oder notwendige Anwenderreaktionen beinhalten.

2. Blutbehandlungseinrichtung nach Anspruch 1, **dadurch gekennzeichnet, dass** Mittel vorgesehen sind, um den selektierten Datensatz bzw. dessen Funktionsablauf auf einem Bildschirm graphisch darzustellen.

3. Blutbehandlungseinrichtung nach einem der Ansprüche 1 - 2, **dadurch gekennzeichnet, dass** der Datensatz (Kₙ) außer dem Funktionsablauf auch Parameter der Bildschirmanzeige umfasst.

4. Blutbehandlungseinrichtung nach einem der Ansprüche 1 - 3, **dadurch gekennzeichnet, dass** ein separater Rechner (45) vorgesehen ist, der die Programmbibliothek enthält und mit der Steuereinrichtung des Blutbehandlungsgerätes (35) kommuniziert.

5. Blutbehandlungseinrichtung nach Anspruch 4, **dadurch gekennzeichnet, dass** zur Kommunikation zwischen der Programmbibliothek und dem Blutbehandlungsgerät (35) ein portabler Datenträger vorgesehen ist.

6. Blutbehandlungseinrichtung nach Anspruch 4, **dadurch gekennzeichnet, dass** der separate Rechner (45) über ein Kommunikationsnetzwerk mit mehreren Blutbehandlungsgeräten (35) verbunden ist.

7. Blutbehandlungseinrichtung nach Anspruch 4, **dadurch gekennzeichnet, dass** auf dem separaten Rechner (45) ein Modell des Blutbehandlungsgerätes (35) läuft, in welches Parameter eingebbar sind und das einen Funktionsablauf mit diesem Parametern simuliert, wobei die Datensätze (Kₙ) einzelner Funktionsabläufe in die Programmbibliothek einspeicherbar sind.

8. Blutbehandlungseinrichtung nach einem der Ansprüche 1 - 7, **dadurch gekennzeichnet, dass** ein Blutbehandlungsgerät (35) mehrere Datensätze (Kₙ) zur Auswahl enthält.

9. Blutbehandlungseinrichtung nach einem der Ansprüche 1 - 8, **dadurch gekennzeichnet, dass** ein ID-Leser (40) vorgesehen ist, der eine Identifikation eines Anwenders detektiert, wobei in Abhängigkeit hiervon die Steuereinrichtung einen Datensatz (Kₙ) auswählt.

10. Blutbehandlungseinrichtung nach einem der Ansprüche 1 - 9, **dadurch gekennzeichnet, dass** ein ID-Leser (40) vorgesehen ist, der eine Identifikation eines Patienten detektiert, wobei in Abhängigkeit hiervon die Steuereinrichtung einen Datensatz (Kₙ) auswählt.

11. Blutbehandlungseinrichtung nach einem der Ansprüche 1 - 10, **dadurch gekennzeichnet, dass** ein Sensor vorgesehen ist, der das Vorhandensein eines bestimmten Verbrauchsartikels sowie dessen Typ in dem Blutbehandlungsgerät (35) detektiert, wobei in Abhängigkeit hiervon die Steuereinrichtung einen oder mehrere Datensätze auswählt.

12. Blutbehandlungseinrichtung nach einem der Ansprüche 1 - 11, **dadurch gekennzeichnet, dass** Mittel vorgesehen sind, um das Einstellen einer bestimmten Behandlungsart an dem Blutbehandlungsgerät (35) zu detektieren, wobei in Abhängigkeit hiervon die Steuereinrichtung einen oder mehrere Datensätze auswählt.

13. Blutbehandlungseinrichtung nach einem der Ansprüche 1 - 12, **dadurch gekennzeichnet, dass** die Programmbibliothek auf einem Datenträger enthalten ist, dessen Inhalt bei Inbetriebnahme des Blutbehandlungsgerätes (35) in dessen Steuereinrichtung eingegeben wird.

14. Verfahren zum Betrieb eines Blutbehandlungsgerätes (35), das aufweist:
einen Filter (10), der an einen Blutkreislauf (13) und einen Dialysatkreislauf (15) angeschlossen ist,
eine Steuereinrichtung zur Steuerung von Funktionsabläufen,
ein Benutzer- Interface (37), das einen Bildschirm (36) und Eingabemittel aufweist,
**dadurch gekennzeichnet,**
**dass** eine Programmbibliothek eingerichtet wird, die unterschiedliche Funktionsabläufe für den Betrieb des Blutbehandlungsgerätes (35) in Form von Datensätzen (Kₙ) enthält, dass für einen Behandlungsablauf einer dieser Datensätze selektiert wird, und dass zum Erzeugen eines in die Programmbibliothek aufzunehmenden Funktionsablaufs eine Eingabe-Bildschirmfläche generiert wird, die einen vom Anwender interaktiv zu beantwortenden Fragenkatalog (50) enthält, wobei, wenn die Funktionen, die in den Funktionsablauf zu übernehmen sind, ausgewählt sind, die Folgeinformationen für den Funktionsablauf erzeugt und angezeigt werden, wobei die Folgeinformationen eine Vorbereitungszeit, einen Verbrauch von Flüssigkeit oder notwendige Anwenderreaktionen beinhalten.

15. Verfahren nach Anspruch 14, **dadurch gekennzeichnet, dass** der Datensatz (Kₙ) bzw. dessen Funktionsablauf auf dem Bildschirm graphisch dargestellt wird.

16. Verfahren nach Anspruch 14 oder 15, **dadurch gekennzeichnet, dass** in den Datensatz außer dem Funktionsablauf auch Parameter der Bildschirmanzeige eingegeben werden.

17. Verfahren nach einem der Ansprüche 14 - 16, **dadurch gekennzeichnet, dass** die Programmbibliothek in einem separaten Rechner erzeugt wird, der mit den Steuereinrichtungen der Blutbehandlungsgeräte kommunizieren kann.

18. Verfahren nach Anspruch 17, **dadurch gekennzeichnet, dass** die Kommunikation mittels eines portablen Datenträgers erfolgt.

19. Verfahren nach Anspruch 17, **dadurch gekennzeichnet, dass** der separate Rechner über ein Kommunikationsnetzwerk mit mehreren Blutbehandlungsgeräten verbunden wird.

20. Verfahren nach Anspruch 16, **dadurch gekennzeichnet, dass** auf dem separaten Rechner ein Modell des Blutbehandlungsgerätes (35) gebildet wird, in welches Parameter eines Funktionsablaufes eingegeben und auf einen Funktionsablauf mit diesen Parametern simuliert, wobei die Datensätze einzelner Funktionsabläufe in die Programmbibliothek eingespeichert werden.

21. Verfahren nach einem der Ansprüche 14 - 20, **dadurch gekennzeichnet, dass** in ein Dialysegerät mehrere Datensätze zur Auswahl eingegeben werden.

22. Verfahren nach einem der Ansprüche 14 - 21, **dadurch gekennzeichnet, dass** ein ID-Leser eine Identifikation eines Anwenders detektiert und die Steuereinrichtung in Abhängigkeit hiervon einen Datensatz auswählt.

23. Verfahren nach einem der Ansprüche 14 - 21, **dadurch gekennzeichnet, dass** ein ID-Leser eine Identifikation eines Patienten detektiert und die Steuereinrichtung in Abhängigkeit hiervon einen Datensatz auswählt.

24. Verfahren nach einem der Ansprüche 14 - 23, **dadurch gekennzeichnet, dass** ein Sensor das Vorhandensein eines bestimmten Verbrauchsartikels in dem Blutbehandlungsgerät (35) detektiert und die Steuereinrichtung in Abhängigkeit hiervon einen oder mehrere Datensätze auswählt.

25. Verfahren nach einem der Ansprüche 14 - 23, **dadurch gekennzeichnet, dass** das Einstellen einer bestimmten Behandlungsart an dem Blutbehandlungsgerät (35) detektiert wird und die Steuereinrichtung in Abhängigkeit hiervon einen oder mehrere Datensätze auswählt.

26. Verfahren nach einem der Ansprüche 14 - 25, **dadurch gekennzeichnet, dass** die auf einem Datenträger enthaltene Programmbibliothek bei Inbetriebnahme des Blutbehandlungsgeräts (35) automatisch in dessen Steuereinrichtung eingelesen wird.

## Claims

1. Blood treatment apparatus for extracorporeal blood treatment, comprising at least one blood treatment device (35) which has:
a filter (10) connected to a blood circulation (13) and a dialysis circuit (15),
a control system for controlling functional sequences,
a user interface (37) having a screen (36) and input means,
**characterised in that**
a program library is provided which contains different functional sequences in the form of data records (Kₙ) for the operation of the blood treatment device (35), it being possible to select a respective data record which is to be activated for a treatment process, and **in that** in order to generate a functional sequence which is to be included in the program library, a device is provided which generates a screen surface containing a list of questions (50) to be answered interactively by the user, wherein when the functions which are to be transferred into the functional sequence have been selected, the follow-on information for the functional sequence is generated and displayed, the follow-on information containing a preparation time, a consumption of liquid or required user reactions.

2. Blood treatment apparatus according to claim 1, **characterised in that** means are provided to graphically display the selected data record or the functional sequence thereof on a screen.

3. Blood treatment apparatus according to any one of claims 1 to 2, **characterised in that** the data record (Kₙ) also comprises parameters of the screen display in addition to the functional sequence.

4. Blood treatment apparatus according to any one of claims 1 to 3, **characterised in that** a separate computer (45) is provided which contains the program library and communicates with the control system of the blood treatment device (35).

5. Blood treatment apparatus according to claim 4, **characterised in that** a portable data carrier is provided for the communication between the program library and the blood treatment device (35).

6. Blood treatment apparatus according to claim 4, **characterised in that** the separate computer (45) is connected to a plurality of blood treatment devices (35) via a communication network.

7. Blood treatment apparatus according to claim 4, **characterised in that** running on the separate computer (45) is a model of the blood treatment device (35) into which parameters can be input and which simulates a functional sequence with these parameters, it being possible to store the data records (Kₙ) of individual functional sequences in the program library.

8. Blood treatment apparatus according to any one of claims 1 to 7, **characterised in that** one blood treatment device (35) contains a plurality of data records (Kₙ) for selection.

9. Blood treatment apparatus according to any one of claims 1 to 8, **characterised in that** an ID reader (40) is provided which detects an identification of a user, the control system selecting a data record (Kₙ) subject thereto.

10. Blood treatment apparatus according to any one of claims 1 to 9, **characterised in that** an ID reader (40) is provided which detects an identification of a patient, the control system selecting a data record (Kₙ) subject thereto.

11. Blood treatment apparatus according to any one of claims 1 to 10, **characterised in that** a sensor is provided which detects the presence of a particular article of use as well as the type thereof in the blood treatment device (35), the control system selecting one or more data records subject thereto.

12. Blood treatment apparatus according to any one of claims 1 to 11, **characterised in that** means are provided to detect the setting of a particular type of treatment on the blood treatment device (35), the control system selecting one or more data records subject thereto.

13. Blood treatment apparatus according to any one of claims 1 to 12, **characterised in that** the program library is contained on a data carrier, the content of which, during the activation of the blood treatment device (35), is input into the control system of the blood treatment device.

14. Method for operating a blood treatment device (35) which comprises:
a filter (10) connected to a blood circulation (13) and a dialysis circuit (15),
a control system for controlling functional sequences,
a user interface (37) having a screen (36) and input means,
**characterised in that**
a program library is set up containing different functional sequences in the form of data records (Kₙ) for the operation of the blood treatment device (35), **in that** one of these data records is selected for a treatment process, and **in that** in order to generate a functional sequence which is to be included in the program library, an input screen surface is generated which contains a list of questions (50) to be answered interactively by the user,
wherein when the functions which are to be transferred into the functional sequence have been selected, the follow-on information for the functional sequence is generated and displayed, the follow-on information containing a preparation time, a consumption of liquid or required user reactions.

15. Method according to claim 14, **characterised in that** the data record (Kₙ) or the functional sequence thereof is displayed graphically on the screen.

16. Method according to claim 14 or claim 15, **characterised in that** parameters of the screen display are also input into the data record in addition to the functional sequence.

17. Method according to any one of claims 14 to 16, **characterised in that** the program library is generated in a separate computer which can communicate with the control systems of the blood treatment devices.

18. Method according to claim 17, **characterised in that** the communication takes place by means of a portable data carrier.

19. Method according to claim 17, **characterised in that** the separate computer is connected to a plurality of blood treatment devices via a communication network.

20. Method according to claim 16, **characterised in that** formed on the separate computer is a model of the blood treatment device (35) into which parameters of a functional sequence are input and which simulates a functional sequence with these parameters, the data records of individual functional sequences being stored in the program library.

21. Method according to any one of claims 14 to 20, **characterised in that** a plurality of data records is input into a dialysis device for selection.

22. Method according to any one of claims 14 to 21, **characterised in that** an ID reader detects an identification of a user and the control system selects a data record subject thereto.

23. Method according to any one of claims 14 to 21, **characterised in that** an ID reader detects an identification of a patient and the control system selects a data record subject thereto.

24. Method according to any one of claims 14 to 23, **characterised in that** a sensor detects the presence of a particular article of use in the blood treatment device (35) and the control system selects one or more data records subject thereto.

25. Method according to any one of claims 14 to 23, **characterised in that** the setting of a particular type of treatment on the blood treatment device (35) is detected and the control system selects one or more data records subject thereto.

26. Method according to any one of claims 14 to 25, **characterised in that** the program library contained on a data carrier is automatically read into the control system of the blood treatment device (35) when the blood treatment device is activated.

## Revendications

1. Dispositif de traitement de sang pour le traitement extracorporel du sang, avec au moins un appareil de traitement de sang (35) qui comprend :
un filtre (10) qui est branché à un circuit de sang (13) et à un circuit de dialysat (15),
un dispositif de commande pour la commande de séquences de fonctions,
une interface utilisateur (37) qui comprend un écran (36) et un moyen d'entrée,
**caractérisé**
**en ce qu'**il est prévu une bibliothèque de programmes qui contient différentes séquences de fonctions pour le fonctionnement de l'appareil de traitement de sang (35) sous forme de jeux de données (Kn), dans lequel chaque jeu de données à activer pour une séquence de traitement peut être sélectionné, et en ce que, pour la génération d'une séquence de fonctions à enregistrer dans la bibliothèque de programmes, il est prévu un dispositif qui génère une surface d'écran qui contient un catalogue de questions (50) à répondre de manière interactive par l'utilisateur, dans lequel, lorsque les fonctions, qui sont reprises dans la séquence de fonctions, sont choisies, les informations suivantes sont créées et affichées pour la séquence de fonctions, dans lequel les informations suivantes incluent un temps de préparation, une consommation de liquide ou des réactions nécessaires de l'utilisateur.

2. Dispositif de traitement de sang selon la revendication 1, **caractérisé en ce que** sont prévus des moyens pour représenter graphiquement sur un écran le jeu de données sélectionné ou sa séquence de fonctions.

3. Dispositif de traitement de sang selon l'une quelconque des revendications 1 et 2, **caractérisé en ce que** le jeu de données (Kn) comporte en plus de la séquence de fonctions également des paramètres de l'affichage sur l'écran.

4. Dispositif de traitement de sang selon l'une quelconque des revendications 1 à 3, **caractérisé en ce qu'**il est prévu un calculateur séparé (45) qui contient la bibliothèque de programmes et communique avec le dispositif de commande de l'appareil de traitement de sang (35).

5. Dispositif de traitement de sang selon la revendication 4, **caractérisé en ce qu'**il est prévu un support de données portable pour la communication entre la bibliothèque de programmes et l'appareil de traitement de sang (35).

6. Dispositif de traitement de sang selon la revendication 4, **caractérisé en ce que** le calculateur séparé (45) est relié, par le biais d'un réseau de communication, à plusieurs appareils de traitement de sang (35).

7. Dispositif de traitement de sang selon la revendication 4, **caractérisé en ce que**, sur le calculateur séparé (45), est exécuté un modèle de l'appareil de traitement de sang (35) dans lequel des paramètres peuvent être entrés et qui simule une séquence de fonctions avec ces paramètres, dans lequel les jeux de données (Kn) de différentes séquences de fonctions peuvent être stockés dans la bibliothèque de programmes.

8. Dispositif de traitement de sang selon l'une quelconque des revendications 1 à 7, **caractérisé en ce qu'**un appareil de traitement de sang (35) contient plusieurs jeux de données (Kn) à choisir.

9. Dispositif de traitement de sang selon l'une quelconque des revendications 1 à 8, **caractérisé en ce qu'**il est prévu un lecteur d'identification (40) qui détecte une identification d'un utilisateur, dans lequel le dispositif de commande choisit en fonction de celle-ci un jeu de données (Kn).

10. Dispositif de traitement de sang selon l'une quelconque des revendications 1 à 9, **caractérisé en ce qu'**il est prévu un lecteur d'identification (40) qui détecte une identification d'un patient, dans lequel le dispositif de commande choisit en fonction de celle-ci un jeu de données (Kn).

11. Dispositif de traitement de sang selon l'une quelconque des revendications 1 à 10, **caractérisé en ce qu'**il est prévu un capteur qui détecte dans l'appareil de traitement de sang (35) la présence d'un certain article consommable ainsi que son type, dans lequel, en fonction de ceux-ci, le dispositif de commande choisit un ou plusieurs jeux de données.

12. Dispositif de traitement de sang selon l'une quelconque des revendications 1 à 11, **caractérisé en ce qu'**il est prévu des moyens pour détecter le réglage d'une certaine sorte de traitement sur l'appareil de traitement de sang (35), dans lequel, en fonction de cela le dispositif de commande choisit un ou plusieurs jeux de données.

13. Dispositif de traitement de sang selon l'une quelconque des revendications 1 à 12, **caractérisé en ce que** la bibliothèque de programmes est contenue sur un support de données dont le contenu est entré lors de la mise en service de l'appareil de traitement de sang (35) dans le dispositif de commande de ce dernier.

14. Procédé de fonctionnement d'un appareil de traitement de sang (35), qui comprend :
un filtre (10) qui est relié à un circuit de sang (13) et à un circuit de dialysat (15),
un dispositif de commande pour la commande de séquences de fonctions,
une interface utilisateur {37) qui comprend un écran (36) et un moyen d'entrée, **caractérisé**
**en ce qu'**il est installé une bibliothèque de programmes qui contient différentes séquences de fonctions pour le fonctionnement de l'appareil de traitement de sang (35) sous forme de jeux de données (Kn), en ce qu'un de ces jeux de données est sélectionné, pour un processus de traitement, et en ce que pour la création d'une séquence de fonctions à enregistrer dans la bibliothèque de programmes est générée une surface d'écran d'entrée qui contient un catalogue de questions (50) à répondre de manière interactive par l'utilisateur, dans lequel, lorsque les fonctions, qui sont à appliquer dans la séquence de fonctions, sont choisies, les informations suivantes sont créées et affichées pour la séquence de fonctions, dans lequel les informations suivantes incluent un temps de préparation, une consommation de liquide ou des réactions nécessaires de l'utilisateur.

15. Procédé selon la revendication 14, **caractérisé en ce que** le jeu de données (Kn) ou sa séquence de fonctions est représenté graphiquement sur l'écran.

16. Procédé selon la revendication 14 ou 15, **caractérisé en ce que**, dans le jeu de données, en plus de la séquence de fonctions sont entrés également des paramètres de l'affichage sur l'écran.

17. Procédé selon l'une quelconque des revendications 14 à 16, **caractérisé en ce que** la bibliothèque de programmes est créée dans un calculateur séparé qui peut communiquer avec les dispositifs de commande de l'appareil de traitement de sang.

18. Procédé selon la revendication 17, **caractérisé en ce que** la communication est effectuée au moyen d'un support de données portable.

19. Procédé selon la revendication 17, **caractérisé en ce que** le calculateur séparé est relié par le biais d'un réseau de communication à plusieurs appareils de traitement de sang.

20. Procédé selon la revendication 16, **caractérisé en ce que**, sur le calculateur séparé est formé un modèle de l'appareil de traitement de sang (35) dans lequel des paramètres d'une séquence de fonctions sont entrés et qui simule une séquence de fonctions avec ces paramètres, dans lequel les jeux de données de différentes séquences de fonctions fonctionnelles sont stockés dans la bibliothèque de programmes.

21. Procédé selon l'une quelconque des revendications 14 à 20, **caractérisé en ce que** plusieurs jeux de données sont entrés aux fins de sélection dans un appareil de dialyse.

22. Procédé selon l'une quelconque des revendications 14 à 21, **caractérisé en ce qu'**un lecteur d'identification détecte une identification d'un utilisateur et le dispositif de commande sélectionne en fonction de celle-ci un jeu de données.

23. Procédé selon l'une quelconque des revendications 14 à 21, **caractérisé en ce qu'**un lecteur d'identification détecte une identification d'un patient et le dispositif de commande sélectionne en fonction de celle-ci un jeu de données.

24. Procédé selon l'une quelconque des revendications 14 à 23, **caractérisé en ce qu'**un capteur détecte la présence d'un certain article consommable dans l'appareil de traitement de sang (35) et le dispositif de commande sélectionne en fonction de celle-ci un ou plusieurs jeux de données.

25. Procédé selon l'une quelconque des revendications 14 à 23, **caractérisé en ce que** le réglage d'une certaine sorte de traitement est détecté sur l'appareil de traitement de sang (35) et le dispositif de commande sélectionne en fonction de cela un ou plusieurs jeux de données.

26. Procédé selon l'une quelconque des revendications 14 à 25, **caractérisé en ce que** la bibliothèque de programmes contenue sur un support de données est écrite, lors de la mise en service de l'appareil de traitement de sang (35), automatiquement dans le dispositif de commande de celui-ci.
